# EUROPEAN PATENT SPECIFICATION

(11) **EP 1 242 091 B1**
(45) Date of publication and mention of the grant of the patent: **23.07.2008**
(21) Application number: 00983101.7
(22) Date of filing: 31.10.2000
(51) Int. Cl.: A61K 31/55, A61P 25/08

(54) **USE OF PHARMACEUTICAL COMPOSITIONS COMPRISING OXCARBAZEPINE IN THE FASTED STATE**
VERWENDUNG VON PHARMAZEUTISCHEN ZUBEREITUNGEN ENTHALTEND OXCARBAZEPIN IM NÜCHTERNZUSTAND
UITILISATION DES COMPOSITIONS PHARMACEUTIQUES COMPRENANT DU OXCARBAZEPINE A JEUN

(30) Priority: 02.11.1999 GB 9925962
(43) Date of publication of application: 25.09.2002
(73) Proprietor: Novartis AG, 4056 Basel (CH); Novartis Pharma GmbH, 1230 Wien (AT)
(72) Inventor: LANG, Steffen, CH-4153 Reinach (CH)
(74) Representative: Gros, Florent
(86) International application number: PCT/EP2000/010764
(87) International publication number: WO 2001/032183

(56) References cited:
- EP-A- 0 646 374
- "Oxcarbazepine approved for partial seizures" AMERICAN JOURNAL OF HEALTH-SYSTEM PHARMACY, vol. 57, no. 5, 1 March 2000 (2000-03-01), pages 414-417, XP002188045 usa
- DEGEN P.H. ET AL.: "THE INFLUENCE OF FOOD ON THE DISPOSITION OF THE ANTIEPILEPTIC OXCARBAZEPINE AND ITS MAJOR METABOLITES IN HEALTHY VOLUNTEERS" BIOPHARMACEUTICS & DRUG DISPOSITION , vol. 15, no. 6, August 1994 (1994-08), pages 519-526, XP002188046
- HELI JUNG ET AL.: "Influence of food on bioavailability of carbazepine" PHARMACEUTICAL RESEARCH, vol. 11 , no. 10 Supl., October 1994 (1994-10), page s219 XP002188047

## Description

This invention is concerned with formulations comprising oxcarbazepine.

Oxcarbazepine (10,11-dihydro-10-oxo-5H-dibenz(b,f)azepine-5-carboximide) is an agent of first choice in the treatment of convulsions.

The bioavailability and bioequivalence of a drug substance depend on its physicochemical properties such as solubility and pharmacokinetic properties, e.g. site, rate and extent of absorption. Further, it is known that food induces changes in the physiology of the gastrointestinal (Gl) tract. These changes can result *i.a* in delays in gastric emptying, stimulation of bile flow and changes in pH. Food can also alter lumenal metabolism and physically or chemically interact with a drug substance. It is not surprising therefore that food can affect the bioavailability of a drug substance.

Degen et al, Biopharmaceutics and Drug Deposition, Vol 15, 519-526 (1994) discusses the effect of food on oxcarbazepine.

Heli Jung et al, Pharmaceutical Research, vol 11, 219 (1994) provides an insight in to the metabolic pathways that can be used to metabolise oxcarbazepine.

WO 98/35861 discusses new formulations for oxcarbazepine.

The effects of food are complicated and difficult to predict and will depend, for example on the nature of the meal, e.g. nutrient content, fluid volume, caloric content, and temperature. It follows that the presence of a food effect for a given drug substance can only be determined after exhaustive testing.

It is undesirable if the bioavailability of a drug substance differs depending upon whether a patient is in a fed or fasted state. This will at least be inconvenient to the patient who will have to time its medication relative to the taking of meals. However, this may be of particular concern for an anti-convulsant drug like oxcarbazepine, which if dosed inconsistently as a result, for example, of the patient receiving food, may lead to adverse events, for example seizures.

In the case of oxcarbazepine which is a neutral molecule having poor solubility in aqueous media, e.g. simulated gastric media, which once dissolved is essentially fully absorbed, and which is administered in relatively large doses (e.g. from 300 up to 600mg oxcarbazepine) one would expect that the increased residence time of the oxcarbazepine in the GI tract and the increased concentration of bile salts in the GI tract both associated with the ingestion of food, would act as an aid to solubility and therefore affect bioavailability.

The above considerations are supported in that oxcarbazepine formulations currently marketed are sold with labeling that draws attention to a food effect and specifies dosing instructions relative to meals, e.g. with a labeling stating that tablets should be taken during or after a meal with liquid.

It is surprising therefore that it was discovered that an oral dosage form of oxcarbazepine may be administered to a patient without regard to the condition of the patient, i.e. whether the patient is in a fed or fasted state.

Accordingly, the invention provides a use of oxcarbazepine, as claimed in claim 1.

Oral dosage forms according to the invention represent a considerable advantage over currently marketed formulations in that they are more convenient and/or safer for patients to use.

The term "food effect" as used herein is intended to mean that the bioavailability of oxcarbazepine in the fed state differs from the bioavailability in the fasted state. The presence or absence of a food effect may be quantified by making Area Under the Curve (AUC) and/or C ₘₐₓ measurements according to methods well known in the art. Typically AUC measurements and C ₘₐₓ measurements are made by taking timed biological fluid samples and plotting the serum concentration of oxcarbazepine (or the active agent thereof) against time. The values obtained represent a number of values taken from subjects across a patient population and are therefore expressed as mean values expressed over the entire patient population. By comparing the mean AUC and/or C ₘₐₓ values, one can determine whether oxcarbazepine exhibits a food effect.

Food effect studies may be conveniently carried out on an adequate number of healthy volunteers, the number being sufficient to generate sufficient data for appropriate statistical assessment to be made. Preferably the number of subjects should not be less than 12.

To study the effect of food on the bioavailability of oxcarbazepine formulations one may use any conventional study design known in the art, for example a randomised, balanced single-dose, two-treatment, two-period, two-sequence crossover design. Analysis may be carried out using, e.g. SAS PROC GLM, software from the SAS institute, Cary North Carolina.

In quantitative terms, oxcarbazepine may be said to exhibit no food effect if 90% confidence interval (CI) for the ratio of means (population geometric means based on log transformed data) of fed and fasted treatments fall within the interval of 0.8 to 1.25 for AUC or 0.7 to 1.43 for C ₘₐₓ.

Accordingly, the present invention provides the use of an oral dosage form according to the invention wherein the oral dosage form comprising oxcarbazepine has a ratio AUC _{ted/fasted} after single dosing of 0.8 to 1.25 or the ratio Cmax _{fed/tasted} after single dosing of 0.7 to 1.43.

By application of the teachings of the present invention oral dosage forms are obtainable providing reduced variability in resorption/bioavailability levels achieved both for individual patients receiving oxcarbazepine therapy as well as between individuals.

A "fed" subject conveniently may be considered as a subject that has fasted for at least 10 hours before receiving a standard FDA recognised high fat meal. The oxcarbazepine may then be administered with water shortly after completion of the meal, e.g. within 5 minutes thereof. Preferably no food should be taken for a period of, e.g. 4 hours after receiving oxcarbazepine although small quantities of water may be permitted after, e.g. 2 hours after receiving the oxcarbazepine.

A "fasted" subject conveniently may receive oxcarbazepine with water after at least 10 hours fasting. Thereafter, no food may be taken for a period of, e.g. 4 hours although small quantities of water may be taken after, e.g. 2 hours after receiving oxcarbazepine.

A standard FDA high fat meal as referred to hereinabove may comprise any meal that would be expected to provide maximal perturbation due to the presence of food in the Gl tract. Said high fat meal typically may comprise 50% of its caloric value in fat. A representative example may be 2 eggs fried in butter, 2 strips of bacon, 2 slices toast with butter, 4 ounces fried potato, and 8 ounces milk.

Oral dosage forms according to the invention rapidly disintegrate upon ingestion to provide the oxcarbazepine rapidly for dissolution. Thereafter oxcarbazepine dissolves within a time period such that there is no resultant food effect. The absence of a food effect for oral dosage forms containing oxcarbazepine may be due in part to properties intrinsic to the drug substance, but it may also be due in part to formulation aspects, e.g. the quality of the drug substance, e.g. the size of drug particles, particle size distribution and the like and/or nature and quality of the excipients, and/or processing factors, e.g. the method of forming the oral dosage form. In order to ensure that each manufacturing batch of oral dosage form according to the invention is consistent in its properties, it is desirable to have a parameter which is easily measurable. In this regard, the applicant has found a level C in vitro/in vivo dissolution rate correlation for oral dosage forms of oxcarbazepine. Thus, in vitro dissolution data may be an important control of the quality of the manufactured oral dosage forms.

Accordingly, preferred oral dosage forms according to the invention display in vitro dissolution rates of not less than 70% dissolved after 30 minutes and not less than 80% dissolved after 60 minutes, as measured by the below-mentioned dissolution test.

Therefore, in a further aspect the present invention provides the use of an oral dosage form according to the invention wherein the oral dosage form shows an oxcarbazepine in vitro dissolution rate of not less than 70% dissolved after 30 minutes.

Oxcarbazepine is rapidly and almost completely absorbed upon dissolution. Furthermore, in *vivo* dissolution is rapid compared with absorption and metabolism of oxcarbazepine. It follows that *in vitro* dissolution data may not only be a reliable measure of quality assurance, but may also be predictive of the bioavailability characteristics of formulations containing same, for example formulations having certain *in vitro* dissolution profiles may be taken as exhibiting no food effect.

Particularly preferred formulations according to the invention diplay *in vitro* dissolution rates of at least 90% dissolved after 30 minutes.

*In vitro* dissolution rates may be measured according to a dissolution test method based on the paddle test methodology set forth in the US Pharmacopoeia, The National Formulary 18 (1995) at page 1791-1792, duly modified such that the dissolution medium is an aqueous solution of sodium dodecyl sulphate (SDS), preferably at a concentration of 0.3 to 1.0% sodium dodecyl sulphate in 900ml water. The concentration of SDS may be determined by the size of the tablet used in the dissolution test, thus for a 150mg tablet one may use 0.3% SDS; for a 300mg dose one may use 0.6% SDS; and for a 600mg dose one may use 1.0%SDS. The paddle speed is preferably set at 60 rpm and the temperature set at 37°C.

Oral dosage forms according to the invention are useful for their anti-convulsive action and may be used as monotherapy or adjunctive therapy in the control, prevention or treatment of seizures, e.g. primary generalized tonic-clonic seizures and partial seizures, with or without secondary generalization, e.g. resulting from the onset of epilepsy, status epilepticus, cerbro-vascular disorders, head injury and alcohol withdrawal.

The exact dose of oxcarbazepine and the particular formulation to be administered depend on a number of factors, e.g. the condition to be treated, the desired duration of treatment and the rate of release of the oxcarbazepine. For example, the amount of oxcarbazepine required and the rate of release thereof may be determined by *in vitro* or *in vivo* techniques, determining how long a particular active agent concentration in the blood plasma remains at an acceptable level to achieve a therapeutic effect.

Preferred regimens according to the invention include for monotherapy, 150 to 600 mg, e.g. 300 mg twice-a-day. Doses from 1200 to 2400 mg/day are tolerated. Preferred regimens for adjunctive therapy include a starting dose of 300mg/day. Doses from 600 to 2400mg/day are tolerated.

Accordingly, in a further aspect the present invention provides the use of an oral dosage form according to the invention wherein the oral dosage form comprises from 300 to 600mg oxcarbazepine.

Whereas oral dosage forms according to the invention may be in the form of solid oral dosage forms, e.g. capsules, powders, or suspensions, it is preferred if oral dosage forms are in the form of tablets.

Conventionally a tablet may comprise a core of oxcarbazepine mixed and compressed with standard tableting excipients.

Oxcarbazepine and its method of preparation are well known in.the art. Its manufacture and therapeutic use are described in German Auslegeschrift 2011 087. A commercially advantageous process of manufacturing oxcarbazepine is disclosed in European Patent Application No. 0 028 028.

Preferably, oxcarbazepine in finely ground form and having a median particle size of approximately 2 to 12 microns, more particularly 4 to 12 microns, still more preferably 4 to 10 microns, and having a maximum residue on a 40 micron sieve of up to 5%, e.g. 2%, is used.

Accordingly, the present invention provides the use according to claim 1 of an oral dosage form according to the invention wherein the oral dosage form either comprises oxcarbazepine in finely ground form and having a median particle size of approximately 4 to 10 microns, and having a maximum residue on a 40 micron sieve of up to 5% or wherein the oral dosage form is other than an oral dosage form which comprises oxcarbazepine in finely ground form and having a median particle size of approximately 4 to 10 microns, and having a maximum residue on a 40 micron sieve of up to 5%.

The known particle size analysis methods are suitable for determining the mean particle size, for example particle size measurement using light, for example light-scattering methods or turbidimetric methods, sedimentation methods, for example pipette analysis using an Andreassen pipette, sedimentation scales, photosedimentometers or sedimentation in a centrifugal force field, pulse methods, for example using a Coulter counter, or sorting by means of gravitational or centrifugal force. Those methods are described, *inter alia,* in *Voigt, loc. cit.,* pages 64-79.

In order to produce oxcarbazepine having the preferred particle size, conventional comminution and de-agglomeration techniques may be used, for example grinding in an airjet mill or impact mill, a ball mill, vibration mill, mortar mill or pin mill.

Oral dosage forms according to the invention, may contain, in addition to oxcarbazepine, conventional excipients depending on the exact nature of the formulation. Suitable categories of excipients include binders, flavourings, diluents, lubricants, thickening agents and glidants.

In the case of a tablet formulation according to the invention suitable excipients may be employed in the core. Suitable excipients include pulverulent fillers having flow-conditioning properties, for example talcum, silicon dioxide, for example synthetic amorphous anhydrous silicic acid of the Syloid^{®} type (Grace), for example SYLOID 244 FP, microcrystalline cellulose, for example of the Avicel^{®} type (FMC Corp.), for example of the types AVICEL PH101, 102, 105, RC581 or RC 591, Emcocel^{®} type (Mendell Corp.) or Elcema^{®} type (Degussa), carbohydrates, such as sugars, sugar alcohols, starches or starch derivatives, for example lactose, dextrose, saccharose, glucose, sorbitol, mannitol, xylitol, potato starch, maize starch, rice starch, wheat starch or amylopectin, tricalcium phosphate, calcium hydrogen phosphate or magnesium trisilicate, binders, such as gelatin, tragacanth, agar, alginic acid, cellulose ethers, for example methylcellulose, carboxymethylcellulose or hydroxypropylmethylcellulose, polyethylene glycols or ethylene oxide homopolymers, especially having a degree of polymerisation of approximately from 2.0 x 10³ to 1.0 x 10⁵ and an approximate molecular weight of about from 1.0 x 10⁵ to 5.0 x 10⁶, for example excipients known by the name Polyox^{®} (Union Carbide), polyvinylpyrrolidone or povidones, especially having a mean molecular weight of approximately 1000 and a degree of polymerisation of approximately from 500 to 2500, and also agar or gelatin, surface-active substances, for example anionic surfactants of the alkyl sulfate type, for example sodium, potassium or magnesium n-dodecyl sulfate, n-tetradecyl sulfate, n-hexadecyl sulfate or n-octadecyl sulfate, of the alkyl ether sulfate type, for example sodium, potassium or magnesium n-dodecyloxyethyl sulfate, n-tetradecyloxyethyl sulfate, n-hexadecyloxyethyl sulfate or n-octadecyloxyethyl sulfate, or of the alkanesulfonate type, for example sodium, potassium or magnesium n-dodecanesulfonate, n-tetradecanesulfonate, n-hexadecanesulfonate or n-octadecanesulfonate, non-ionic surfactants of the fatty acid polyhydroxy alcohol ester type, such as sorbitan monolaurate, monooleate, monostearate or monopalmitate, sorbitan tristearate or trioleate, polyoxyethylene adducts of fatty acid polyhydroxy alcohol esters, such as polyoxyethylene sorbitan monolaurate, monooleate, monostearate, monopalmitate, tristearate or trioleate, polyethylene glycol fatty acid esters, such as polyoxyethyl stearate, polyethylene glycol 400 stearate, polyethylene glycol 2000 stearate, especially ethylene oxide/propylene oxide block polymers of the Pluronics^{®} (BWC) or Synperonic^{®} (ICI) type.

Preferably tablets are coated.

Accordingly, in one aspect the present invention provides the use of an oral dosage form avccording to the invention wherein the oral dosage form is a coated tablet.

Oxcarbazepine displays a tendency towards discolouration upon storage and coatings, e.g. a single or a double coating may be beneficial in masking any discolouration. Accordingly the invention provides in another of its aspects, a solid oral dosage form as hereinabove described which is stable to discolouration. Preferably oral dosage forms that are stable to discolouration remain so stable for at least 3 years storage at a temperature of 25 °C and 60% r.h..

It may be beneficial in masking discolouration, to use colouring agents, e.g pigments in oral dosage forms according to the invention. In the case of a tablet, colouring agents may be mixed with oxcarbazepine and tabletting excipients in the core or they may alternatively be placed solely in a coating composition, or both in the core and in the coating composition.

However, having regard to requirements of regulatory authorities concerning the use of high concentrations of certain colouring agents in pharmaceutical preparations, it is preferable to keep the concentration of colouring agent as low as possible and in any case below the level permitted by regulatory authorities. For example, the FDA imposes a limit on the amount of iron oxide (ferric or ferrous oxide) that may be ingested by a patient which is currently set at 5 mg/day elemental iron.

Further, discolouration is a concentration phenomenon, that is, discolouration is more apparent the higher the concentration of the oxcarbazepine in the oral dosage form. It follows that the amount of pigment necessary to mask discolouration will depend upon the concentration of the active substance in a unit dosage form. As the oral dosage forms according to the invention may contain relatively high concentrations of oxcarbazepine, it is not always possible to mask discolouration by simply adding pigment to the core of a tablet whilst still keeping within the regulatory limits for the given colouring agent.

Surprisingly, the applicant has now found that applying relatively small quantities of colouring agent to a coating surrounding the core of a tablet provides for a desirable masking effect with reduced concentrations of colouring agent which concentrations fall within acceptable limits.

Any of the colouring agents known for use in pharmaceutical preparations are suitable for use in the present invention, see for example "Handbook of Pharmaceutical Excipients, 2nd Edition (1994), Eds. Wade and Weller", at pages 130-134. Suitable colouring agents include titanium dioxide, iron oxide (both ferrous and ferric), preferably Fe₂O₃ optionally in hydrated form.

When a colouring agent is employed, the amounts employed in the coating will depend upon the size of the particular dosage form and the concentration of oxcarbazepine. Preferably the amount of colouring agent employed, e.g. iron oxide is from about 0.1 to 1.6mg per unit dosage form, e.g. tablet, more preferably 0.3mg per unit dosage form to 0.9mg per unit dosage form. The colouring agent has a particularly good masking effect when employed solely in the coating composition.

Suitable coating materials include those materials conventionally used in coating tablets, granules and the like. Preferred coating materials are hydrophilic and permeable to, and/or at least to some extent soluble in, water and intestinal fluids. Any of the coating materials, in particular the elastic coatings described in published European patent application PCT/EP 9800794, incorporated herein by reference, are suitable for the purposes of the present invention.

Coating materials as hereinabove defined may be used in admixture with other excipients, conventional in coating formulations, for example talcum or silicon dioxide, for example synthetic amorphous silicic acid of the Syloid^{®} type (Grace), for example SYLOID 244 FP, or wetting agents, for example the afore-mentioned polyethylene glycols or sorbates.

The coating materials may comprise additional excipients, for example plasticisers e.g. triethyl citrate, e.g. Citroflex^{®} (Pfizer), triacetin, various phthalates, e.g. diethyl or dibutyl phthalate, mixed mono- or di-glycerides of the Myvacet^{®} type (Eastman), for example MYVACET 9-40, the polyethylene glycols mentioned hereinbefore, for example having a molecular weight of approximately from 6000 to 8000, and also ethylene oxide/propylene oxide block copolymers of the Pluronic^{®} (BASF) or Synperonic^{®} (ICI) type, pulverulent mould release agents, for example magnesium trisilicate, starch or synthetic amorphous silicic acid of the SYLOID type, for example SYLOID 244 FP.

Oral dosage forms according to the invention in the form of coated tablets contain a high proportion of oxcarbazepine for a given unit dosage form. Preferably, coated tablets according to the invention contain up to 75% by weight of oxcarbazepine, more particularly 65 to 75%, e.g. 65 to 73% by weight.

Commercially available forms of oxcarbazepine contain up to 75% by weight of oxcarbazepine. However, these forms are uncoated tablets. Furthermore, the high concentration of oxcarbazepine in commercially available forms was achieved by means of a low-shear ethanolic granulation process. However, industrial processes advantageously should not employ organic solvents. Still further, given that the tablets in the present invention may undergo an additional coating step, a wet granulation technique would apparently have the disadvantage that the tablet core would have to dry or be substantially dry before carrying out the subsequent coating step thus slowing considerably the manufacturing process. On the other hand, if the tablet core is not sufficiently dry before coating, further drying of the tablet core may compromise the integrity of the coating.

Unfortunately, the applicant found that it was with great practical difficulty that highly concentrated tablets could be made using conventional dry granulation and compaction techniques as compression of the tablet mass (due in part to the intrinsic properties of oxcarbazepine, in particular the milled substance, and in part due to the large amount of excipient required to make large dosage forms) was not possible without recourse to impractically high compression forces for industrial purposes.

After exhaustive testing, the applicant found that highly concentrated (with respect to oxcarbazepine) coated tablets could be formed using a wet granulation technique which tablets could be formed within an acceptable time for manufacturing purposes and with a coating, the integrity of which remains intact.

Granules are produced in a manner known *per se,* for example using aqueous granulation methods known for the production of "built-up" granules or "broken-down" granules.

Methods for the formation of built-up granules operate continuously and comprise, for example, simultaneously spraying the granulation mass with granulation solution and drying, for example in a drum granulator, in pan granulators, on disc granulators, in a fluidised bed, by spray-drying or spray-solidifying, or operate discontinuously, for example in a fluidised bed, in a batch mixer or in a spray-drying drum.

Preferred are methods for the production of broken-down granules, which can be carried out discontinuously and in which the granulation mass first forms a wet aggregate with the granulation solution, which aggregate is then comminuted or formed into granules of the desired particle size using known granulation methods, the granules then being dried. Suitable granulators include, for example the Alexander granulator set forth in Example 1.

The granulation mass consists of comminuted, preferably ground, active ingredient and the excipients mentioned above, for example pulverulent fillers, such as microcrystalline cellulose of the AVICEL type. AVICEL PH 102 is especially suitable. Depending on the method used, the granulation mass may be in the form of a premix or may be obtained by mixing the active ingredient into one or more excipients or mixing the excipients into the active ingredient. The wet granules are preferably dried, for example in the described manner by spray drying or in a fluidised bed.

Compression to form tablet cores may be carried out in conventional tabletting machines, for example EK-0 Korsch eccentric tabletting machines. The tablet cores may be of various shapes, for example round, oval, oblong, cylindrical etc., and various sizes, depending on the amount of active ingredient employed.

Oxcarbazepine having mean particle size of approximately from 4 to 12 µm, e.g. 6 to 8 µm, with a maximum residue on a 40 µm sieve of 2 % is particularly suited to the process hereinabove described.

Tablet cores formed by a method according to the invention may be coated.

Preferably, the coating composition is dissolved or suspended in water in the desired quantity ratio. If desired, excipients, such as polyethylene glycol, may be added. The solution or dispersion may be sprayed onto the tablet cores together with other excipients, for example talcum or silicon dioxide, for example SYLOID 244 FP, using known methods such as spray-coating in a fluidised bed, for example using the Aeromatic, Glatt, Wurster or Hüttlin (ball coater) system, or also in a coating pan in accordance with the methods known by the names Accela Cota or immersion coating.

Preferably, an aqueous dispersion comprising hydroxypropylmethylcellulose (cellulose HPMC) and pigments is sprayed on to the core of a tablet using conventional spray coating techniques.

The following Examples illustrate the invention.

### Example 1

### Formulations

| **Tablet core** | [mg] | [mg] |
|---|---|---|
| TRILEPTAL AS extra fine | 300.0 | 600.0 |
| Cellulose HPM 603 | 8.4 | 16.8 |
| Microcrystalline Cellulose | 65.6 | 131.2 |
| Colloidal anhydrous silica | 1.6 | 3.2 |
| Magnesium stearate | 4.4 | 8.8 |
| Crosspovidone | 20.0 | 40.0 |
| Core Weight | 400.0 | 800.0 |

| **Coating** | | |
|---|---|---|
| Cellulose HPM 603 | 7.351 | 11.946 |
| Iron(II) oxide (yellow) 17268 | 0.499 | 0.811 |
| Polyethylene glycol (PEG) 8000 | 1.331 | 2.162 |
| Talcum | 5.323 | 8.649 |
| Titanium dioxide | 1.497 | 2.432 |
| **Coating Weight** | **16.000** | **26.000** |

Mix the TRILEPTAL, cellulose HPM 603 (binder) and a portion (approximately half) of the microcrystalline cellulose (binder, filler, disintegration-promoting excipient) in a mixer, preferably in a high-speed mixer, e.g. DIOSNA, LOEDIGE, FIELDER or GLATT. Add water to the mixture and knead, preferably in a high-speed mixer until an adequate consistency is achieved. Alternatively, the HPM 603 may be dissolved in the water, beforehand. Granulate the wet granules using an ALEXANDER Reibschnitzler, QUADRO-COMILL and dry in a fluidised bed (AEROMATIC, GLATT). Add the remainder of the microcrystalline cellulose, AEROSIL 200 (flow conditioner) and crospovidone (disintegrator) to the dry granules and mix in a comminuter (FREWITT, QUADRO-COMILL, FITZMILL). Finally, add magnesium stearate (lubricant) and mix (STOECKLIN container mixer, VRIECO mixer). Alternatively, the lubricant may be added directly to the comminuted material. Compress the final mixture to form TRILEPTAL tablets (eccentric press, rotary press: KILIAN, KORSCH, FETTE, MANESTY).

Coat the tablets with an aqueous preparation consisting of cellulose HPM 603 (film former), iron oxide yellow 17268 (pigment), PEG 8000 (plasticiser for the film former), talcum (anti-adhesive agent, covering agent) and titanium dioxide (covering agent) in a rotating coating pan (ACCELA-COTA, GLATT, DRIACOATER, DUMOULIN). Alternatively, it is possible to use, for example, fluidised-bed or air-suspension apparatus for the coating process (AEROMATIC, GLATT, FREUND, HUETTLIN).

### Example 2: Food Effect

20 healthy male volunteers are dosed with 1200 mg trileptal (2X 600 mg) on each of two occasions. On the first occasion the volunteers are dosed after 12 hours lasting, and on the other occasion the volunteers are dosed within 15 minutes after eating a high-fat breakfast consisting of 2 small packets of cereal (50 g), 150 g of semi-skimmed milk, 200 mL orange juice, 2 slices of wholemeal toast, 10 g low fat spread and 20 g jam. The trileptal tablets are taken with 200 ml water. Blood samples are taken prior to dosing and at time intervals of 1 hour at steady-state and at the following time points after single oral dosing: 0.5, 1, 2, 3, 4, 5, 6, 8, 10, 12, 24, 32, 48, 56, 72 hours post-dosing. Plasma concentrations of the active agent are determined using a HPLC assay. For each volunteer under each dosing condition, the Area Under the drug plasma Concentration-vs- time curve (AUC) is determined.

The average ratio of AUC(fed)/AUC(fasted) is calculated as 0.98 with lower and upper limit 90% confidence levels of 0.94 and 1.02 respectively after single administration. The average ratio of AUC(fed)/AUC(fasted) is calculated as 0.99 with lower and upper limit 90% confidence levels of 0.96 and 1.03 respectively after single administration.

These results demonstrate that food has no effect on the bioavailability of the trileptal formulation.

## Claims

1. The use of oxcarbazepine, which has a median particle size of from 2 to 12 micrometer and a maximum residue on a 40 micrometer sieve of up to 5%, for the preparation of a medicament for the control, prevention or treatment of seizures in a subject in need thereof, which medicament is administered orally to the said subject, which subject is **characterized by** being in the fasted state.

2. The use according to claim 1, **characterized in that** the oxcarbazepine has a median particle size of from 4 to 12 micrometer.

3. The use according to claim 1, **characterized in that** the oxcarbazepine has a median particle size of from 4 to 10 micrometer.

4. The use according to any one of claims 1 to 3, **characterized in that** the oxcarbazepine has a maximum residue on a 40 micrometer sieve of up to 2%.

5. The use according to claim 1, **characterized in that** the oxcarbazepine has a median particle size of from 6 to 8 micrometer and a maximum residue on a 40 micrometer sieve of up to 2%.

6. The use according to any one of claims 1 to 5, **characterized in that** the said medicament comprises the oxcarbazepine in an amount of from 300 mg to 600 mg.

7. The use according to any one of claims 1 to 6, **characterized in that** the said medicament is a coated tablet.

8. The use according to any one of claims 1 to 7, **characterized in that** the said medicament shows an in-vitro dissolution rate, according to which at least 70 % of the oxcarbazepine are dissolved after 30 minutes.

9. The use according to any one of claims 1 to 8, **characterized in that** the said medicament shows an in-vitro dissolution rate, according to which at least 90 % of the oxcarbazepine are dissolved after 30 minutes.

10. The use according to any one of claims 1 to 9, **characterized in that** the said medicament shows an oxcarbazepine ratio AUC_{fed/fasted} after single dosing of from 0.8 to 1.25.

11. The use according to any one of claims 1 to 10, **characterized in that** the said medicament shows an oxcarbazepine ratio Cmax_{fed/fasted} after single dosing of from 0.7 to 1.43.

12. The use according to any one of claims 1 to 11, **characterized in that** the said medicament is used in monotherapy.

13. The use according to any one of claims 1 to 11, **characterized in that** the said medicament is used in adjunctive therapy.

## Patentansprüche

1. Verwendung von Oxcarbazepin, das eine mittlere Teilchengröße von 2 bis 12 µm und einen maximalen Rückstand auf einem 40 µm Sieb von bis zu 5 % hat, zur Herstellung eines Arzneimittels für die Bekämpfung, Prävention oder Behandlung von Anfällen bei einem behandlungsbedürftigen Subjekt, wobei dieses Arzneimittel dem Subjekt oral verabreicht wird und dieses Subjekt **dadurch gekennzeichnet ist, dass** es sich im nüchternen Zustand befindet.

2. Verwendung nach Anspruch 1, **dadurch gekennzeichnet, dass das** Oxcarbazepin eine mittlere Teilchengröße von 4 bis 12 µm hat.

3. Verwendung nach Anspruch 1, **dadurch gekennzeichnet, dass** das Oxcarbazepin eine mittlere Teilchengröße von 4 bis 10 µm hat.

4. Verwendung nach einem der Ansprüche 1 bis 3, **dadurch gekennzeichnet, dass** das Oxcarbazepin einen maximalen Rückstand auf einem 40 µm Sieb von bis zu 2 % hat.

5. Verwendung nach Anspruch 1, **dadurch gekennzeichnet, dass** das Oxcarbazepin eine mittlere Teilchengröße von 6 bis 8 µm und einen maximalen Rückstand auf einem 40 µm Sieb von bis zu 2 % hat.

6. Verwendung nach einem der Ansprüche 1 bis 5, **dadurch gekennzeichnet, dass** im genannten Arzneimittel das Oxcarbazepin in einer Menge von 300 mg bis 600 mg vorliegt.

7. Verwendung nach einem der Ansprüche 1 bis 6, **dadurch gekennzeichnet, dass** das Arzneimittel eine beschichtete Tablette ist.

8. Verwendung nach einem der Ansprüche 1 bis 7, **dadurch gekennzeichnet, dass** das Arzneimittel eine in vitro Auflösungsrate zeigt, entsprechend welcher wenigstens 70 % des Oxcarbazepins nach 30 min in Lösung gegangen sind.

9. Verwendung nach einem der Ansprüche 1 bis 8, **dadurch gekennzeichnet, dass** das Arzneimittel eine in vitro Auflösungsrate zeigt, entsprechend welcher wenigstens 90 % des Oxcarbazepins nach 30 min in Lösung gegangen sind.

10. Verwendung nach einem der Ansprüche 1 bis 9, **dadurch gekennzeichnet, dass** das Arzneimittel ein Oxcarbazepinverhältnis AUC_{nicht nüchtern/nüchtern} nach einer Einzeldosierung von 0,8 bis 1,25 zeigt.

11. Verwendung nach einem der Ansprüche 1 bis 10, **dadurch gekennzeichnet, dass** das Arzneimittel ein Oxcarbazepinverhältnis Cmax_{nicht nüchtern/nüchtern} nach einer Einzeldosierung von 0,7 bis 1,43 zeigt.

12. Verwendung nach einem der Ansprüche 1 bis 11, **dadurch gekennzeichnet, dass** das Arzneimittel in einer Monotherapie angewandt wird.

13. Verwendung nach einem der Ansprüche 1 bis 11, **dadurch gekennzeichnet, dass** das Arzneimittel in einer adjunktiven Therapie angewandt wird.

## Revendications

1. Utilisation d'oxcarbazépine, qui présente une taille moyenne de particules de 2 à 12 micromètres et un pourcentage maximal de particules retenues sur un tamis de calibre 40 micromètres pouvant atteindre 5 %, pour la préparation d'un médicament destiné à contrôler, à prévenir ou à traiter les crises chez un sujet en ayant besoin, lequel médicament est administré par voie orale audit sujet, lequel sujet est **caractérisé par** le fait d'être à jeun.

2. Utilisation selon la revendication 1, **caractérisée en ce que** l'oxcarbazépine présente une taille moyenne de particules de 4 à 12 micromètres.

3. Utilisation selon la revendication 1, **caractérisée en ce que** l'oxcarbazépine présente une taille moyenne de particules de 4 à 10 micromètres.

4. Utilisation selon l'une quelconque des revendications 1 à 3, **caractérisée en ce que** l'oxcarbazépine présente un pourcentage maximal de particules retenues sur un tamis de calibre 40 micromètres pouvant atteindre 2%.

5. Utilisation selon la revendication 1, **caractérisée en ce que** l'oxcarbazépine présente une taille moyenne de particules de 6 à 8 micromètres et un pourcentage maximal de particules retenues sur un tamis de calibre 40 micromètres pouvant atteindre 2 %.

6. Utilisation selon l'une quelconque des revendications 1 à 5, **caractérisée en ce que** ledit médicament comprend l'oxcarbazépine en une quantité de 300 mg à 600 mg.

7. Utilisation selon l'une quelconque des revendications 1 à 6, **caractérisée en ce que** ledit médicament est un comprimé enrobé.

8. Utilisation selon l'une quelconque des revendications 1 à 7, **caractérisée en ce que** ledit médicament présente une vitesse de dissolution *in vitro* selon laquelle au moins 70 % de l'oxcarbazépine est dissous au bout de 30 minutes.

9. Utilisation selon l'une quelconque des revendications 1 à 8, **caractérisée en ce que** ledit médicament présente une vitesse de dissolution *in vitro* selon laquelle au moins 90 % de l'oxcarbazépine est dissous au bout de 30 minutes.

10. Utilisation selon l'une quelconque des revendications 1 à 9, **caractérisée en ce que** ledit médicament présente un rapport AUC _{nourri/à jeun} pour l'oxcarbazépine, après l'administration d'une dose, de 0,8 à 1,25.

11. Utilisation selon l'une quelconque des revendications 1 à 10, **caractérisée en ce que** ledit médicament présente un rapport Cmax _{nourri/à jeun} pour l'oxcarbazépine, après l'administration d'une dose, de 0,7 à 1,43.

12. Utilisation selon l'une quelconque des revendications 1 à 11, **caractérisée en ce que** ledit médicament est utilisé en monothérapie.

13. Utilisation selon l'une quelconque des revendications 1 à 11, **caractérisée en ce que** ledit médicament est utilisé en traitement d'appoint.
